# EUROPEAN PATENT SPECIFICATION

(11) **EP 2 531 112 B1**
(45) Date of publication and mention of the grant of the patent: **22.11.2017**
(21) Application number: 11702125.3
(22) Date of filing: 28.01.2011
(51) Int. Cl.: A61B 10/04, A61B 10/02, A61B 1/00

(54) **NESTING ENDOSCOPIC ULTRASOUND GUIDED BIOPSY DEVICE**
VERSCHACHTELTE ENDOSKOPISCHE ULTRASCHALLGEFÜHRTE BIOPSIEVORRICHTUNG
IMBRICATION DE DISPOSITIF ENDOSCOPIQUE DE BIOPSIE GUIDÉ PAR ULTRASONS

(30) Priority: 05.02.2010 US 301833 P
(43) Date of publication of application: 12.12.2012
(73) Proprietor: Boston Scientific Scimed, Inc., Maple Grove, MN 55311-1566 (US)
(72) Inventor: RYAN, Shawn, Upton, MA 01568 (US); BURNIM, Kayla, Somerville, MA 02144 (US)
(74) Representative: Vossius & Partner Patentanwälte Rechtsanwälte mbB
(86) International application number: PCT/US2011/022853
(87) International publication number: WO 2011/097125

(56) References cited:
- US-A1- 2003 208 136
- US-A1- 2004 260 199
- US-A1- 2005 124 977
- US-A1- 2006 116 605
- US-A1- 2006 189 891
- US-A1- 2009 299 217
- US-B1- 6 228 049

## Description

### Background

Needle biopsies are common for the diagnosis and the staging of disease. In particular, in endoscopic ultrasound-guided fine needle aspiration (EUS-FNA), a biopsy needle is imaged under ultrasound so that the physician is able to visualize a position of the needle in relation to target tissue. Thus, EUS-FNA procedures ensure that the correct tissue is sampled while minimizing risk to the patient. Although EUS-FNA is a highly sensitive and specific procedure, in certain situations it may be difficult to acquire a satisfactory sample. For example, in some situations the needle must be advanced to the target site within the body along a tortuous path (e.g., through a natural body lumen) making it difficult to employ stiffer, large diameter needles. In addition, such larger gauge needles may collect tissue samples including a large amount of blood or other fluids which may interfere with analysis of the sampled tissue.

US 6 228 049 B1 discloses an introducer guide for introducing medical items into an internal tissue of a patient. The guide includes a cannula defining a lumen sized to receive the item and a lateral opening. A ramp is disposed within the lumen adjacent and inclined toward the lateral opening.

US 2005/0124977 A1 discloses a catheter device having an elongated tubular member with a proximal end and a distal end, and a needle disposed near the distal end.

US 2009/0299217 A1 discloses an apparatus for taking tissue samples and the like in the context of use of a kind of examination or treatment telescope in the treatment of humans or animals, wherein the apparatus for taking tissue samples and the like is provided with a receptacle for accommodating tissue samples and the like, and wherein, at the end of biopsy forceps, coupling means are formed for exchangeable attachment of a tool for use in connection with the biopsy forceps.

US 2006/0116605 A1 discloses a rotating fine needle for core tissue sampling.

### Summary of the Invention

A biopsy device as recited in the independent claim is provided. The dependent claims define embodiments.

The present invention is directed to a biopsy device for insertion to a target site within a living body, comprising a first needle including a first longitudinal element extending from a first element proximal end to a tissue piercing first element distal end and, the first longitudinal element defining a first lumen extending therethrough from a proximal opening at the first element proximal end to a distal opening at the first element distal end, the first longitudinal element being sufficiently flexible to be inserted to a target site along a tortuous path within a natural body lumen and a second needle including a second longitudinal element extending from a proximal end to a tissue piercing second element distal end, the second longitudinal element being sized to be slidably received in the first lumen and defining a second lumen extending therewithin to a tissue receiving opening at the second element distal end, the second longitudinal element being sufficiently flexible to be inserted to a target site along a tortuous path within a natural body lumen.

### Brief Description of the Drawings

Fig. 1 shows a side view of a system according to an exemplary embodiment of the present invention; and
Fig. 2 shows a side view of a coupling of first and second needle devices of the system of Fig. 1;
Fig. 3 shows a cross-sectional side view of a portion of nested first and second longitudinal elements;
Fig. 4 shows a side view of a distal end of the system of Fig. 1; and
Fig. 5 shows an enlarged side view of the distal end of Fig. 4.

### Detailed Description

The present invention may be further understood with reference to the following description and the appended drawings, wherein like elements are referred to with the same reference numerals. The present invention relates to endoscopic devices and, in particular, relates to endoscopic biopsy devices. An exemplary embodiment of the present invention is directed to an endoscopic biopsy device including a first needle inserted into the body to a target area and a second needle insertable through a lumen of the first needle to access the target area and collect a tissue sample therefrom. It will be understood by those of skill in the art that the nesting of the second needle within the first needle permits the target area to be sampled multiple times while minimizing trauma to the target area and to the tissue surrounding the path along which the device is inserted to the target site. It should be noted that the terms distal and proximal, as used herein, are intended to indicate a direction away from (distal) and toward (proximal) a user of the device.

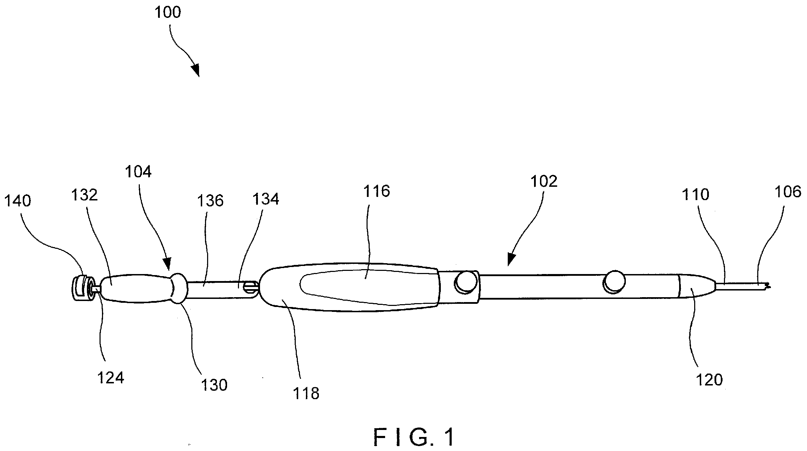

As shown in Figs. 1 - 5, a system 100 according to an exemplary embodiment of the present invention comprises a first needle 102 and a second needle 104 connectable to one another. The first needle 102 includes a first longitudinal element 106 defining a lumen 114 extending therethrough from an open proximal end 110 to an open distal end 112. The first lumen 114 is sized and shaped to accommodate a second longitudinal element 108 of the second needle 104. The first longitudinal element 106 may be flexible along a length thereof such that the first longitudinal element 106 may be inserted into the body along a tortuous path (e.g., within a body lumen) to reach a target area within the body.

A proximal end 110 of the first longitudinal element 106 is coupled to a first handle 116 which extends from a proximal end 118 to a distal end 120 and includes a channel (not shown) extending therethrough sized to slidably receive the first longitudinal element 106 therethrough. The length of the first longitudinal element 106 is selected so that, when inserted through the channel of the first handle 116 with the proximal end 110 of the first longitudinal element 106 coupled thereto in a desired configuration, a distal end 112 extends distally out of the first handle 116 by a desired distance (e.g., a distance equal to a length of an endoscope to be coupled to the first handle 116 plus a desired projection of the needle distally beyond a distal end of the endoscope. The channel is arranged so that, when the first handle 116 is coupled to an endoscope in a desired configuration, the channel of the first handle 116 aligns with a working channel of the endoscope so that the first needle 102 is slid therethrough to an insertion/withdrawal position with the distal end 112 received within the distal end of the endoscope. As discussed below, after the endoscope has been advanced to a target location, the first needle 102 may be moved distally to a deployed position in which the distal end 112 extends distally out of the distal end of the endoscope (e.g., to penetrate target tissue).

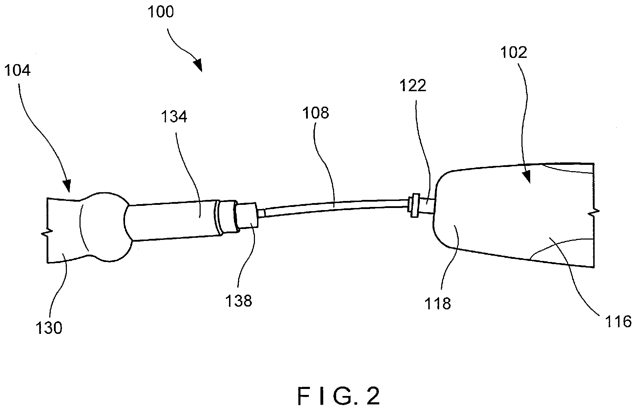

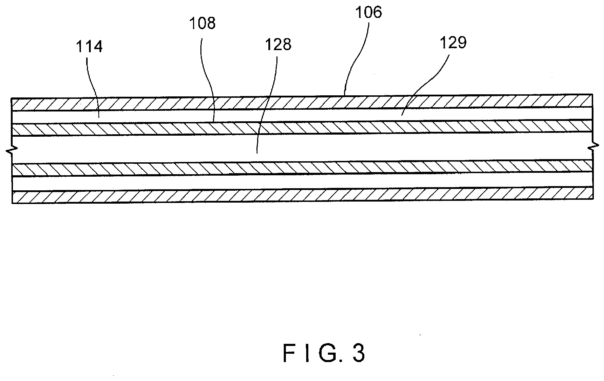

As shown in Fig. 2, the first handle 116 may further include a luer fitting 122 for coupling to the second needle 104. The second longitudinal element 108 extends longitudinally from a proximal end 124 to a distal end 126 and includes a lumen 128 extending through at least a portion of a length thereof. As shown in Fig. 3, an outer diameter of the second longitudinal element 108 is smaller than an inner diameter of the first lumen 114 of the first longitudinal element 106 such that the second longitudinal element 108 may be slidably inserted through the first lumen 114. For example, in a preferred embodiment, the first longitudinal element 106 may be a 19 gauge needle while the second longitudinal element is a 22 gauge needle. Alternatively, the first longitudinal element 106 may be a 22 gauge needle while the second longitudinal element 108 may be a 25 gauge needle.

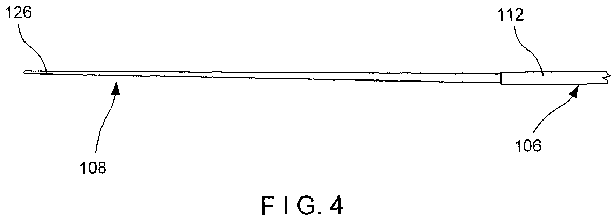

A length of the second longitudinal element 108 is preferably longer than a length of the first longitudinal element 106 such that when the second longitudinal element 108 is inserted into the first lumen 114, the distal end 126 of the second longitudinal element 108 may be extended distally past the distal end 112 of the first longitudinal element 106, as shown in Fig 4 when desired. The proximal end 124 of the second longitudinal element 108 may include a stop 140 that extends radially outward from the second longitudinal element 108 to define a distal-most point to which the second longitudinal element 108 may be inserted into the first longitudinal element 106. A sample of target tissue may be collected within the distal end 126 of the lumen 128. However, it will be understood by those of skill in the art that the second longitudinal element 108 may include any other tissue collecting mechanism such as, for example, a lateral groove or a distal facing opening in the distal end 126.

A second handle 130 which may be attached to the proximal end 124 of the second longitudinal element 108 extends from a proximal end 132 to a distal end 134 and includes a channel 136 within which the extending therethrough. The channel 136 is sized and shaped to slidably receive the second longitudinal element 108 therethrough such that the second longitudinal element 108 may be inserted through the proximal end 132 of the handle until the distal end 126 of the second longitudinal element extends distally past the distal end 134 of the second handle 130. The stop 140 at the proximal end 124 of the second longitudinal element 108 prevents the proximal end 124 of the second longitudinal element 108 from sliding distally past the proximal end 132 of the second handle 130. The distal end 134 of the second handle 130 of this embodiment includes a coupling element 138 adapted and configured to mate with the luer fitting 122 of the first needle 102 such that the first and second needles 102, 104 may be connected to one another.

The second longitudinal element 108 of the second needle 104 may be inserted through the lumen 114 of the first longitudinal element 106 of the first needle 102 such that the second longitudinal element 108 is "nested" within the first longitudinal element 106. The second longitudinal element 108 may be slid through the lumen 114 until the coupling element 138 of the second needle 104 comes into contact with the luer fitting 122 of the first needle 102. The coupling element 13 and the luer fitting 122 mate with one another such that the first and second needles 102, 104 are coupled to one another. In a preferred embodiment, the luer fitting 122 and the coupling element 138 may be press-fit to one another. It will be understood by those of skill in the art, however, that although the exemplary embodiments describe the luer fitting 122 and the coupling element 138, the first and second needles 102, 104 may be coupled to one another using any known coupling mechanism. Once the first and second needles 102, 104 have been coupled to one another, the second longitudinal element 108 may be repeatedly used to collect tissue samples from the target area.

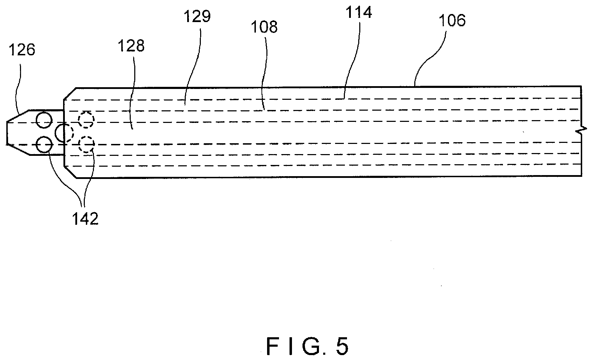

As would be understood by those skilled in the art, suction may be applied to the system 100 in an annular space 129 within the first longitudinal element 106 and outside the second longitudinal element 118 using for example, a Y-connector. In addition, as shown in Fig. 5, the system 100 includes a filtering mechanism formed as a plurality of filter holes 142 at the distal end 126 of the second longitudinal element 108 sized to permit non- targeted materials such as blood and other fluids received in the lumen 128 to be suctioned through the wall of the second longitudinal element 108 into the space 129 between the first and second longitudinal elements 106, 108 while preventing target tissue received in the lumen 128 from passing therethrough. For example, where the non-targeted material is blood, each of the filter holes 142 has a diameter of approximately 8-10 µm allowing red blood cells to flow therethrough while preventing harvested target tissue from passing out. The system 100 may further include a seal about the outer diameter of the second longitudinal element 108 at the distal end 126 that seals the space 129 to ensure that the suctioning force is transferred through the filter holes 142 to the lumen 128.

An exemplary method of use of the system 100 comprises coupling the distal end of the first handle 116 to an endoscope so that the channel extending therethrough aligns with a working channel of the endoscope. The first longitudinal element 106 of the first needle 102 is then slid through the channel of the first handle 116 into the working channel of the endoscope and advanced therethrough until the distal end 112 is in a desired position relative to the distal end of the endoscope (e.g., in the insertion/withdrawal configuration with the distal end 112 received within the distal end of the endoscope). The second needle 104 is then inserted through the second handle 130 into the first needle 102 and passed therethrough until the distal end of the second needle 104 is in a desired position within the first needle 102 (e.g., with the distal end 126 received within the first needle 102 in the insertion/withdrawal configuration). The second handle 130 is then coupled to the first needle 102. The endoscope may then be inserted to a location adjacent to target tissue to be sampled in a known manner. The user then advances the first needle 102 distally out of the endoscope (e.g., via distal advancement of the first handle 116)
to a guide configuration in which the distal end 112 penetrates the target tissue. The first needle 102 is maintained in this position to provide a very accurate guide for the second needle 104 nested therein. The second needle 104 is moved to a tissue capture configuration in which it is advanced distally through the first needle 102 into the target tissue to obtain a first tissue sample. As would be understood by those skilled in the art, in the tissue capture configuration, the second needle 104 is advanced by moving the second handle 130 distally until the second handle 130 comes into contact with the first handle 116 of the first needle 102 and the coupling element 138 of the second needle 104 is coupled to the luer fitting 122 of the first needle 102. The user may then apply suction to the space 129 to draw fluids out of the sampled tissue and the second needle 104 is withdrawn from the first needle 102 so that the tissue sample may be analyzed. Specifically, after the tissue sample has been collected, the coupling element 138 may be released from the luer fitting 122 and the second needle 104 may be drawn proximally to remove the second needle 104 from the first needle 102. If it is desired to take another tissue sample, the second needle 104 is reinserted into the first needle 106 and advanced therethrough back into the target tissue mass to obtain a second sample. Suction may then be applied to the space 129 to draw blood and other fluids out of the lumen 128 via the filter holes 142. The second needle 104 may then be withdrawn from the first needle 102 so that the second tissue sample may be removed for analysis. This procedure may be repeated as often as necessary with each sample being drawn from the same single puncture site defined by the distal end 112 of the first needle 102, thereby minimizing patient trauma.

The second needle 104 may be removed from the first needle 102 by decoupling the luer fitting 122 and the coupling element 138 of the first and second needles 102, 104, respectively. When tissue collection has been completed, the first needle 102 is withdrawn proximally back into the working channel of the endoscope into the insertion /withdrawal configuration and the endoscope and first needle 102 are removed from the body in a conventional manner.

It will be apparent to those of skill in the art that various modifications and variations can be made in the structure and the methodology of the present invention, without departing from the scope of the invention as defined by the claims. Thus, it is intended that the present invention cover the modifications and variations of this invention provided that they come within the scope of the appended claims and their equivalents.

## Claims

1. A biopsy device for insertion to a target site within a living body, comprising:
a first needle (102) including a first longitudinal element (106) extending from a first element proximal end (110) to a first element distal end (112), wherein the first longitudinal element (106) defines a first lumen (114) extending therethrough from a proximal opening at the first element proximal end (110) to a distal opening at the first element distal end (112), and wherein the first longitudinal element (106) is sufficiently flexible along a length thereof to be inserted to a target site along a tortuous path within a natural body lumen and configured to pierce tissue;
a second needle (104) including a second longitudinal element (108) extending from a second element proximal end (124) to a second element distal end (126), wherein the second longitudinal element (108) is sized to be slidably received in the first lumen (114) and defines a second lumen (128) extending therewithin to a tissue receiving opening at the second element distal end (126), and wherein the second longitudinal element (108) is sufficiently flexible to be inserted to a target site along a tortuous path within a natural body lumen and configured to pierce tissue, and
a connector;
wherein an outer diameter of the second longitudinal element (108) is smaller than an inner diameter of the first lumen (114) by an amount selected to create a fluid receiving annular (129) space therebetween when the second longitudinal element (108) is inserted into the first lumen (114),
**characterized in that**
the second longitudinal element (108) includes a filter at a distal end thereof, wherein the filter comprises a plurality of holes (142) in a distal portion of a wall of the second longitudinal element (108) and wherein the holes (142) of the filter are between 8 µm and 10 µm in diameter, the filter thereby permitting non-targeted fluids received in the second lumen (128) to pass therethrough from the second lumen (128) into the fluid receiving annular space (129) while preventing target tissue received in the second lumen (128) from passing therethrough when suction is applied to the fluid receiving annular space (129) within the first longitudinal element (106) and outside the second longitudinal element (108) via the connector.

2. The device of claim 1, further comprising:
a coupling mechanism selectively coupling the first and second longitudinal elements (106, 108) to one another.

3. The device of claim 2, wherein the coupling mechanism includes a first luer fitting (122) at a proximal end (118) of a first handle (116) coupleable to the first longitudinal element (106) and a complimentary coupling element (138) at a distal end (134) of a second handle (130) coupleable to the second longitudinal element (108).

4. The device of claim 3, wherein the first handle (116) includes a first channel extending therethrough sized to slidably receive the second longitudinal (108) element therein so that, when the first handle (116) is coupled to the first longitudinal element (106), insertion of the second longitudinal element (108) into the first channel of the first handle (116) directs the second longitudinal element (108) into the first lumen (114).

5. The device of claim 4, wherein the second handle (130) includes a second channel (136) extending therethrough such that the second channel (136) slidably receives the second longitudinal (108) element therethrough.

6. The device of claim 1, wherein the second longitudinal element (108) is longer than the first longitudinal element (106) such that, when inserted into the first lumen (114) to a tissue penetrating configuration, the distal end (126) of the second longitudinal element (108) extends distally past the distal end (122) of the first longitudinal element (106).

## Patentansprüche

1. Biopsievorrichtung zur Einführung zu einer Zielstelle in einem lebenden Körper, mit:
einer ersten Nadel (102), die ein erstes longitudinales Element (106) umfasst, das sich von einem proximalen Ende (110) des ersten Elements zu einem distalen Ende (112) des ersten Elements erstreckt, wobei das erste longitudinale Element (106) ein erstes Lumen (114) definiert, das sich durch dieses von einer proximalen Öffnung am proximalen Ende (110) des ersten Elements zu einer distalen Öffnung am distalen Ende (112) des ersten Elements erstreckt, und wobei das erste longitudinale Element (106) entlang seiner Länge ausreichend flexibel ist, um entlang eines gewundenen Wegs innerhalb eines natürlichen Körperlumens zu einer Zielstelle eingeführt zu werden, und konfiguriert ist, Gewebe zu durchstechen;
einer zweiten Nadel (104), die ein zweites longitudinales Element (108) umfasst, das sich von einem proximalen Ende (124) des zweiten Elements zu einem distalen Ende (126) des zweiten Elements erstreckt, wobei das zweite longitudinale Element (108) bemessen ist, verschiebbar im ersten Lumen (114) aufgenommen zu werden, und ein zweites Lumen (128) definiert, das sich darin zu einer Gewebeaufnahmeöffnung am distalen Ende (126) des zweiten Elements erstreckt, und wobei das zweite longitudinale Element (108) ausreichend flexibel ist, um entlang eines gewundenen Wegs innerhalb eines natürlichen Körperlumens zu einer Zielstelle eingeführt zu werden, und konfiguriert ist, Gewebe zu durchstechen, und
einem Verbindungsstück;
wobei ein Außendurchmesser des zweiten longitudinalen Elements (108) um einen Betrag kleiner als ein Innendurchmesser des ersten Lumens (114) ist, der so ausgewählt ist, dass dazwischen ein Fluidaufnahme-Ringraum (129) geschaffen wird, wenn das zweite longitudinale Element (108) in das erste Lumen (114) eingeführt wird,
**dadurch gekennzeichnet, dass**
das zweite longitudinale Element (108) einen Filter an seinem distalen Ende aufweist, wobei der Filter in einem distalen Abschnitt einer Wand des zweiten longitudinalen Elements (108) mehrere Löcher (142) aufweist und wobei ein Durchmesser der Löcher (142) des Filters zwischen 8 µm und 10 µm beträgt, wodurch der Filter in das zweite Lumen (128) aufgenommene, nicht als Ziel gesetzte Fluide dort hindurch vom zweiten Lumen (128) in den Fluidaufnahme-Ringraum (129) gehen lässt, während in das zweite Lumen (128) aufgenommenes Zielgewebe daran gehindert wird, dort hindurch zu gehen, wenn eine Saugwirkung auf den Fluidaufnahme-Ringraum (129) innerhalb des ersten longitudinalen Elements (106) und außerhalb des zweiten longitudinalen Elements (108) über das Verbindungsstück angewendet wird.

2. Vorrichtung nach Anspruch 1, die ferner aufweist:
einen Kopplungsmechanismus, der das erste und zweite longitudinale Element (106, 108) selektiv miteinander koppelt.

3. Vorrichtung nach Anspruch 2, wobei der Kopplungsmechanismus einen ersten Luer-Anschluss (122) an einem proximalen Ende (118) eines ersten Griffs (116), der mit dem ersten longitudinalen Element (106) koppelbar ist, und ein komplementäres Kopplungselement (138) an einem distalen Ende (134) eines zweiten Griffs (130) aufweist, der mit dem zweiten longitudinalen Element (108) koppelbar ist.

4. Vorrichtung nach Anspruch 3, wobei der erste Griff (116) einen ersten Kanal aufweist, der sich dort hindurch erstreckt und der bemessen ist, das zweite longitudinale Element (108) darin aufzunehmen, so dass, wenn der erste Griff (116) mit dem ersten longitudinalen Element (106) gekoppelt wird, ein Einführen des zweiten longitudinalen Elements (108) in den ersten Kanal des ersten Griffs (116) das zweite longitudinale Element (108) in das erste Lumen (114) führt.

5. Vorrichtung nach Anspruch 4, wobei der zweite Griff (130) einen zweiten Kanal (136) aufweist, der sich dort hindurch erstreckt, so dass der zweite Kanal (136) verschiebbar das zweite longitudinale Element (108) dort hindurch aufnimmt.

6. Vorrichtung nach Anspruch 1, wobei das zweite longitudinale Element (108) länger als das erste longitudinale Element (106) ist, so dass sich, wenn es in das erste Lumen (114) zu einer gewebedurchdringenden Konfiguration eingeführt wird, das distale Ende (126) des zweiten longitudinalen Elements (108) distal über das distale Ende (122) des ersten longitudinalen Elements (106) hinaus erstreckt.

## Revendications

1. Dispositif de biopsie destiné à être inséré dans un site cible à l'intérieur d'un corps vivant, comprenant :
une première aiguille (102) comportant un premier élément longitudinal (106) s'étendant d'une extrémité proximale (110) du premier élément à une extrémité distale (112) du premier élément, le premier élément longitudinal (106) définissant une première lumière (114) s'étendant dans celui-ci d'une ouverture proximale à la extrémité proximale (110) du premier élément à une ouverture distale à la extrémité distale (112) du premier élément, et le premier élément longitudinal (106) étant suffisamment flexible sur sa longueur pour être inséré dans un site cible le long d'un chemin sinueux dans une lumière naturelle du corps et étant prévu pour percer les tissus ;
une deuxième aiguille (104) comportant un deuxième élément longitudinal (108) s'étendant d'une extrémité proximale (124) du deuxième élément à une extrémité distale (126) du deuxième élément, le deuxième élément longitudinal (108) étant dimensionné pour pouvoir être reçu de manière coulissante dans la première lumière (114) et définissant une deuxième lumière (128) s'étendant dans celui-ci vers une ouverture de réception de tissu à la extrémité distale (126) du deuxième élément, et le deuxième élément longitudinal (108) étant suffisamment flexible pour être inséré dans un site cible le long d'un chemin sinueux dans une lumière naturelle du corps et étant prévu pour percer les tissus, et
un connecteur ;
le diamètre extérieur du deuxième élément longitudinal (108) étant inférieur au diamètre intérieur de la première lumière (114) d'une valeur sélectionnée pour créer un espace annulaire de réception de fluide (129) intérieur quand le deuxième élément longitudinal (108) est inséré dans la première lumière (114),
**caractérisé en ce que**
le deuxième élément longitudinal (108) comprend un filtre à son extrémité distale, le filtre présentant une pluralité de trous (142) dans une partie distale d'une paroi du deuxième élément longitudinal (108) et les trous (142) du filtre ayant un diamètre compris entre 8 µm et 10 µm, le filtre permettant ainsi le passage de fluides non ciblés reçus dans la deuxième lumière (128), de la deuxième lumière (128) à l'espace annulaire de réception de fluide (129), en empêchant le passage du tissu cible reçu dans la deuxième lumière (128) quand l'espace annulaire de réception de fluide (129) est soumis à une aspiration à l'intérieur du premier élément longitudinal (106) et à l'extérieur du deuxième élément longitudinal (108) par l'intermédiaire du connecteur.

2. Dispositif selon la revendication 1, comprenant en outre :
un mécanisme d'accouplement reliant sélectivement l'un à l'autre le premier élément et le deuxième élément longitudinal (106, 108).

3. Dispositif selon la revendication 2, où le mécanisme d'accouplement comprend un premier raccord Luer (122) à une extrémité proximale (118) d'une première poignée (116) connectable au premier élément longitudinal (106), et un élément d'accouplement complémentaire (138) à une extrémité distale (134) d'une deuxième poignée (130) connectable au deuxième élément longitudinal (108).

4. Dispositif selon la revendication 3, où la première poignée (116) comprend un premier canal s'étendant dans celle-ci, dimensionnée pour recevoir de manière coulissante le deuxième élément longitudinal (108) de sorte que, quand la première poignée (116) est accouplée au premier élément longitudinal (106), l'insertion du deuxième élément longitudinal (108) dans le premier canal de la première poignée (116) entraîne le guidage du deuxième élément longitudinal (108) dans la première lumière (114).

5. Dispositif selon la revendication 4, où la deuxième poignée (130) comprend un deuxième canal (136) s'étendant dans celle-ci de sorte que le deuxième canal (136) reçoit le deuxième élément longitudinal (108) de manière coulissante.

6. Dispositif selon la revendication 1, où le deuxième élément longitudinal (108) est plus long que le premier élément longitudinal (106) de sorte qu'une fois inséré dans la première lumière (114) dans une configuration de pénétration du tissu, l'extrémité distale (126) du deuxième élément longitudinal (108) s'étend distalement au-delà de l'extrémité distale (122) du premier élément longitudinal (106).
